# EUROPEAN PATENT SPECIFICATION

(11) **EP 3 013 390 B1**
(45) Date of publication and mention of the grant of the patent: **03.05.2017**
(21) Application number: 14739289.8
(22) Date of filing: 10.06.2014
(51) Int. Cl.: A61M 5/145, A61M 5/24, A61M 5/142

(54) **DRUG INFUSION DEVICE WITH SAFETY INTERLOCK**
MEDIKAMENTENINFUSIONSVORRICHTUNG MIT SICHERHEITSVERRIEGELUNG
DISPOSITIF DE PERFUSION DE MÉDICAMENT AVEC VERROUILLAGE DE SÉCURITÉ

(30) Priority: 28.06.2013 US 201361840533 P
(43) Date of publication of application: 04.05.2016
(73) Proprietor: Animas Corporation, West Chester, PA 19380 (US)
(72) Inventor: HUTCHINSON, Michael, King of Prussia, Pennsylvania 19406 (US); DIPIETRO, Joseph, Cumming, Georgia 30041 (US)
(74) Representative: Carpmaels & Ransford LLP
(86) International application number: PCT/US2014/041634
(87) International publication number: WO 2014/209591

(56) References cited:
- EP-A1- 0 615 762
- WO-A1-2005/018721
- WO-A1-2012/128699
- US-A1- 2008 312 585

## Description

### FIELD OF THE INVENTION

The present invention relates, in general, to drug delivery devices and, more particularly, to a drug infusion device that may be worn as a patch-style pump configured to deliver medication to a patient in discrete boluses. The disclosed device may receive commands from a remote device via wireless telemetry and includes a safety interlock to lock-out, or block, remote instructions.

### BACKGROUND OF THE INVENTION

The use of drug delivery devices for various types of drug therapy is becoming more common as the automated infusion of a drug may provide more reliable and more precise treatment to a patient.

Diabetes is a major health concern, as it can significantly impede on the freedom of action and lifestyle of persons afflicted with this disease. Typically, treatment of the more severe form of the condition, Type I (insulin-dependent) diabetes, requires one or more insulin injections per day, referred to as multiple daily injections. Insulin is required to control glucose or sugar in the blood, thereby preventing hyperglycemia that, if left uncorrected, can lead to diabetic ketoacidosis. Additionally, improper administration of insulin therapy can result in hypoglycemic episodes, which can cause coma and death. Hyperglycemia in diabetics has been correlated with several long-term effects of diabetes, such as heart disease, atherosclerosis, blindness, stroke, hypertension, and kidney failure.

The value of frequent monitoring of blood glucose as a means to avoid or at least minimize the complications of Type I diabetes is well established. Patients with Type II (non-insulin-dependent) diabetes can also benefit from blood glucose monitoring in the control of their condition by way of diet and exercise. Thus, careful monitoring of blood glucose levels and the ability to accurately and conveniently infuse insulin into the body in a timely manner is a critical component in diabetes care and treatment.

To more effectively control diabetes in a manner that reduces the limitations imposed by this disease on the lifestyle of the affected person, various devices for facilitating blood glucose (BG) monitoring have been introduced. Typically, such devices, or meters, permit the patient to quickly, and with a minimal amount of physical discomfort, obtain a sample of their blood or interstitial fluid that is then analyzed by the meter. In most cases, the meter has a display screen that shows the BG reading for the patient. The patient may then dose theirselves with the appropriate amount, or bolus, of insulin. For many diabetics, this results in having to receive multiple daily injections of insulin. In many cases, these injections are self-administered.

WO 2012/128699 describes and illustrates a reusable auto-injector with distal and proximal housing parts, the proximal housing part being adapted to receive a medicament container including a movable plunger. A threaded plunger rod is arranged within the auto-injector to act on the plunger and is movable in the longitudinal direction of the device. The threaded plunger rod is configured to be driven toward the proximal end of the device by a drive nut. A guide member is rotatably lockable but longitudinally slidable relative to the threaded plunger rod and is configured to be switched by a lock and release mechanism between a locked state when the housing parts are assembled to each other, in which it cannot rotate, and a released state when the housing parts are disconnected, in which it can rotate. As a result, it is said that torsion force acting on the threaded plunger rod when the guide member is in the released state exceeds a tension force such that the threaded plunger rod is urged towards the proximal end of the device, but that the tension force acting on the threaded plunger rod when the guide member is in its locked state exceeds the torsion force, so that the threaded plunger rod is urged towards the distal end of the device. The auto-injector also includes an actuation mechanism which is operably connected to the drive nut so that the drive nut is locked when the guide member is in its released state.

Due to the debilitating effects that abnormal BG levels can have on patients, i.e., hyperglycemia, persons experiencing certain symptoms of diabetes may not be in a situation where they can safely and accurately self-administer a bolus of insulin. Moreover, persons with active lifestyles find it extremely inconvenient and imposing to have to use multiple daily injections of insulin to control their blood sugar levels, as this may interfere or prohibit their ability to engage in certain activities. For others with diabetes, multiple daily injections may simply not be the most effective means for controlling their BG levels. Thus, to further improve both accuracy and convenience for the patient, insulin infusion pumps have been developed.

Insulin pumps are generally devices that are worn on the patient's body, either above or below their clothing. Because the pumps are worn on the patient's body, a small and unobtrusive device is desirable. Therefore, it would be desirable for patients to have a more compact drug delivery device that delivers medication reliably and accurately. Further it would be desirable for such an infusion system to conform to the patient's body when worn, to reduce discomfort and unintentional dislodgement, and offers the flexibility for the patient to choose to operate the pump with or without an infusion set.

It is further desirable that the device be configured to, at least, replace prior art methods for delivering multiple daily injections by including the ability to deliver discrete boluses of medication. Moreover, to remain concealed, it is desirable that the device be fully controllable via remote telemetry and includes means to lock the drug delivery mechanism to avoid delivery as a result of unauthorized telemetry or spurious RF signals.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:
FIGS. 1A and 1B are perspective and cross-sectional perspective views, respectively, of an in-line drive mechanism in which the drive mechanism is in a retracted position;
FIG. 2 is a cross-sectional perspective view of the in-line drive mechanism illustrated in FIGS. 1A and 1B engaged with a plunger that is inserted into a drug reservoir;
FIG. 3 is a cross-sectional perspective view of the in-line drive mechanism illustrated in FIGS. 1A and 1B with the piston extended;
FIGS. 4A and 4B are simplified perspective views of drug delivery devices;
FIGS. 5A - 5C are cross-sectional perspective views of another in-line drive mechanism with the piston in retracted, intermediate and extended positions, respectively; and
FIGS. 6A - 6C are cross-sectional perspective views of yet another in-line drive mechanism with the piston in retracted, intermediate and extended positions, respectively.
FIG. 7 illustrates a perspective view of an infusion pump in which the infusion pump includes an adapter for receiving an infusion set luer connector.
FIG. 8 depicts a perspective view of a medication reservoir cartridge according to the infusion pump of FIG. 7 and including an adapter for receiving a luer connector.
FIG. 9 depicts a cross-sectional view of an insertable component attached to the adapter for receiving a luer connector.
FIGS. 10A and 10B show illustrations of an infusion pump equipped for tethered (FIG. 10A) and untethered (FIG. 10B) deployment.
FIG. 11 illustrates an embodiment of the housing according to an embodiment of the present invention in perspective view.
FIG. 12 shows an embodiment of an infusion device according to the present invention in partial cross-sectional view and an exploded inset of the pusher assembly.
FIG. 13 illustrates an end of the infusion device showing a controller gear and ratchet claw according to an embodiment of the present invention in perspective and partial cross-sectional view.
FIG. 14 illustrates an end of the infusion device showing a controller gear and ratchet claw according to an embodiment of the present invention in cross-sectional view.
FIG. 15 depicts a remote control device configured to control an infusion pump according to an embodiment of the invention via RF telemetry.

### DETAILED DESCRIPTION OF ILLUSTRATIVE EMBODIMENTS OF THE INVENTION

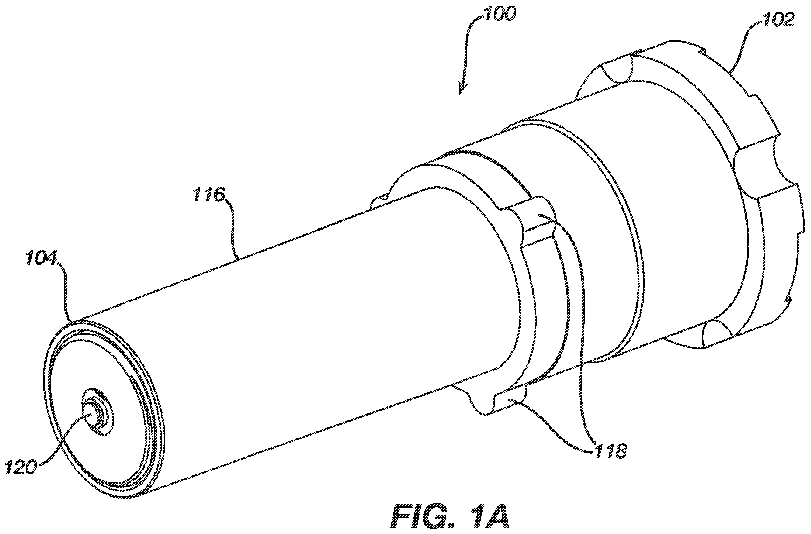

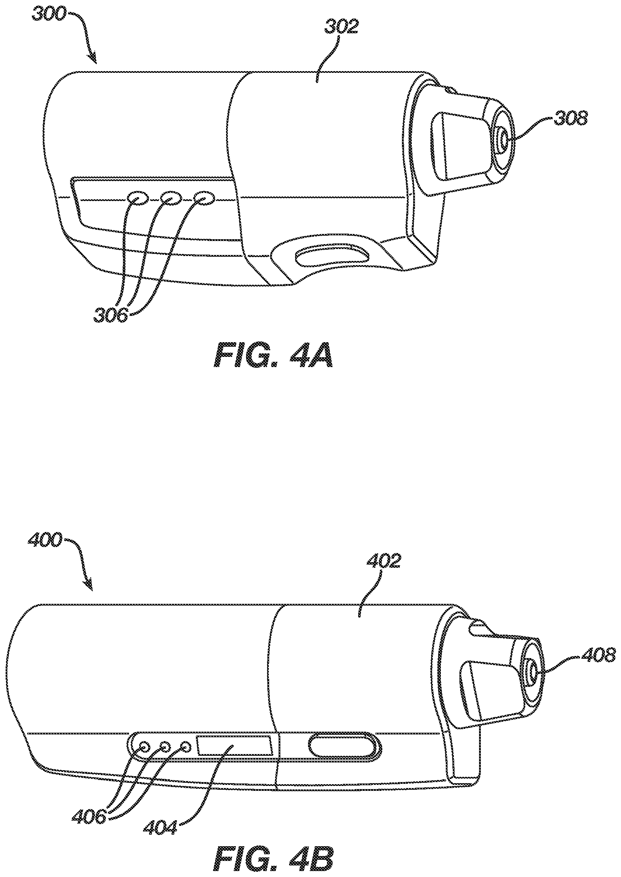

FIGS. 1A - 3 illustrate a drive mechanism 100 of an infusion pump. Generally cylindrical in shape, the drive mechanism 100 includes a proximal end 102, a distal end 104 and a combined motor and gearbox (hereinafter referred to as a "motor 106") operatively coupled to a lead screw 108 that is configured to engage a piston 110. The proximal end 102 of the drive mechanism 100 is compliance mounted (i.e., has a "floating" mount) to an internal surface (not shown) of a housing of a drug delivery device such as, for example, an insulin pump. A compliance mount allows the motor housing to turn slightly in response to high motor torque during motor startup. The distal end 104 of the drive mechanism 100 is configured to engage a plunger 111 that is slidably inserted into a drug reservoir 112 (or cartridge) of a drug delivery device. The drive mechanism 100 is coaxially aligned or "in-line" with the axis of travel of the plunger 111. Drug delivery devices that may be used are illustrated in FIGS. 4A and 4B.

The piston 110 includes a cavity 113 to receive the motor 106 and the lead screw 108 such that the lead screw 108 and at least a portion of the motor 106 are substantially contained within the piston cavity 113 when the piston 110 is in a retracted position. At least a portion of the motor 106 is also substantially contained within a cavity 114 of the lead screw 108 regardless of whether the piston 110 is in the retracted or extended position. In this case, the length of the motor 106 is greater than a diameter of the motor 106. The length of the motor 106 is from about 20 millimeters to about 30 millimeters and the diameter of the motor is from about 5 millimeters to about 10 millimeters. This configuration of the piston 110 , lead screw 108 and motor 106 results in a more compact drug delivery device than with conventional motor configurations which are parallel to the axis of travel of the plunger.

An outer surface 116 of the piston 110 further includes a keying feature 118 that mates with a slot (not shown) in the internal surface of the housing of the drug delivery device. The keying feature 118 prevents rotation of the piston 110 during use of the drive mechanism 100 such that the piston 110 moves only in the axial direction A.

The motor 106 is coupled to and drives a drive shaft 120, which is coupled via a hub to an inner surface 124 of a first end 126 of the lead screw 108. The motor 106 is housed within and is attached to a motor mounting sleeve 128 by at least one dowel pin 130. The motor mounting sleeve 128 prevents the motor 106 from rotating by being keyed (not shown) to a base mount 132 that is attached to an internal surface of the drug delivery device. The base mount 132 radially surrounds the motor mounting sleeve 128 near a proximal end 134 of the motor mounting sleeve 128. A plurality of linear bearings 136 between the motor mounting sleeve 128 and the base mount 132 allow the motor mounting sleeve 128 to "float" axially such that a force sensor 138 can sense a load on the motor 106 when, for example, the infusion line that delivers the drug from the drug reservoir is occluded. The force sensor 138 is coupled to a force sensor contact 140 at the proximal end 134 of the motor mounting sleeve 128.

The lead screw 108 includes external threads 142 that mate with internal threads 144 of the piston 110. Radial bearings 146 that allow rotational movement of the lead screw 108 may be included in a space 148 between a second end 150 of the lead screw 108 and an outer surface 152 of the motor mounting sleeve 128.

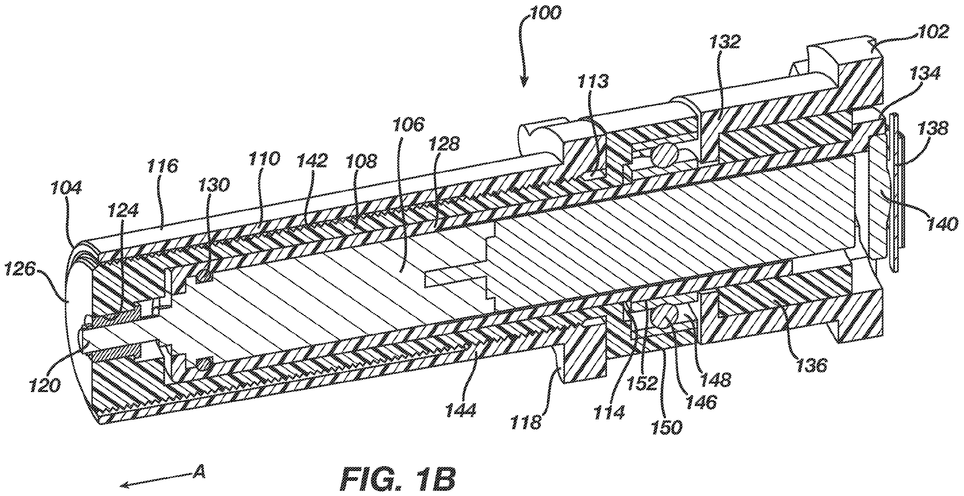

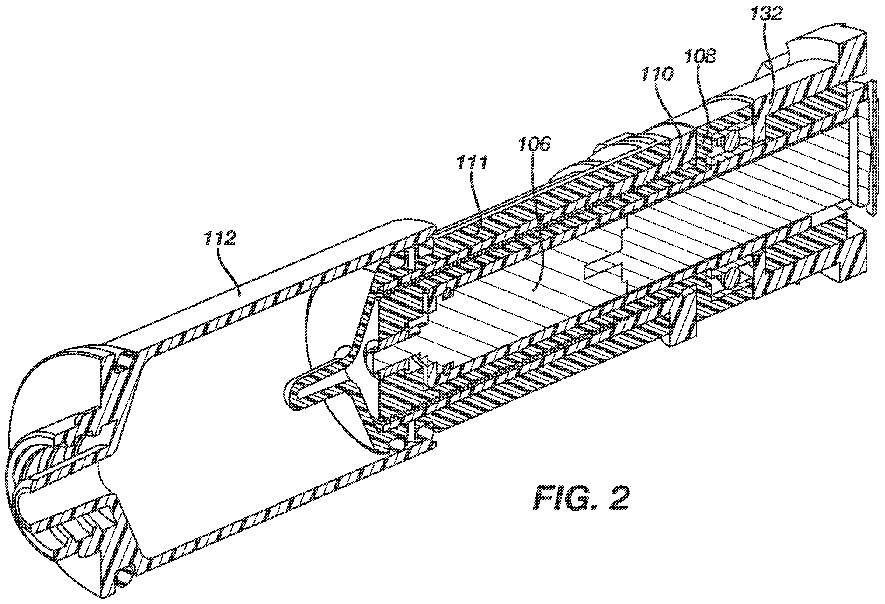

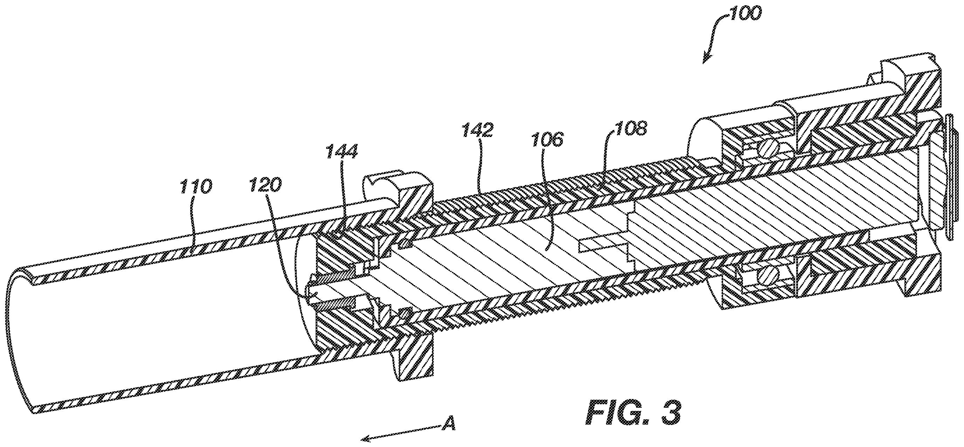

In use, the torque generated from the motor 106 is transferred to the drive shaft 120, which then rotates the lead screw 108. As the lead screw 108 rotates, the external threads 142 of the lead screw 108 engage with the internal threads 144 of the piston 110, causing the piston 110 to move in the axial direction A from a retracted position (see FIG. 1B) to an extended position (see FIG. 3). As the piston 110 moves from the retracted position to the extended position, the distal end of the piston 110 engages the plunger 111 (shown in FIG. 2) such that the drug is delivered from the drug reservoir or cartridge.

Referring to FIGS. 4A and 4B, drug delivery devices 300 and 400 that may be used each include a housing 302 and 402, respectively, a display 404 (not shown in device 300) for providing operational information to the user, a plurality of navigational buttons 306 and 406 for the user to input information, a battery (not shown) in a battery compartment for providing power to drug delivery devices 300 and 400, processing electronics (not shown), drive mechanism 100 for forcing a drug from a drug reservoir through a side port 308 and 408 connected to an infusion set (not shown) and into the body of the user.

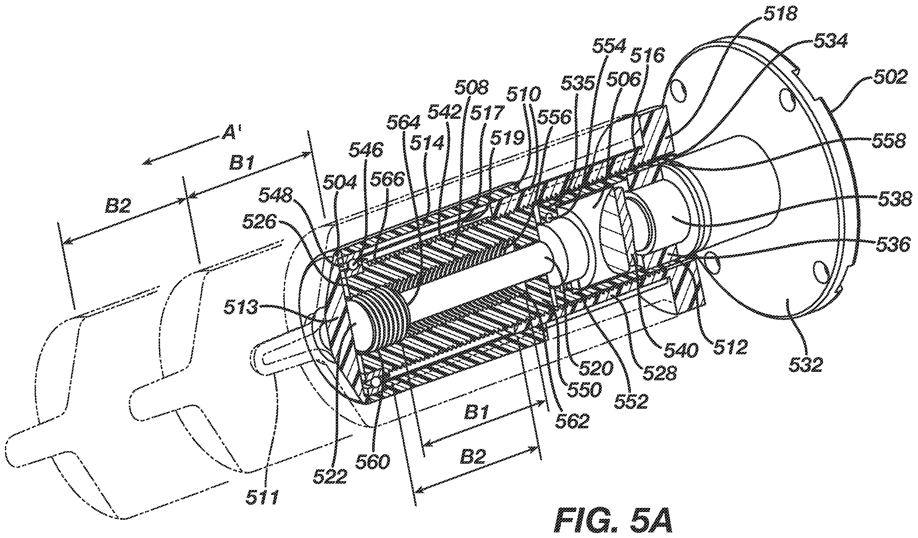

Referring now to FIGS. 5A - 5C, another example is illustrated. The drive mechanism 500 is cylindrical in shape and includes a proximal end 502, a distal end 504 and a motor 506 operatively coupled to a lead screw 508, which is configured to engage a piston 510. The proximal end 502 of the drive mechanism 500 is compliance mounted to an internal surface (not shown) of a housing of a drug delivery device. The distal end 504 of the drive mechanism 500 is configured to engage a plunger 511 that is slidably inserted into a drug reservoir of a drug delivery device. The drive mechanism 500 is coaxially aligned or "in-line" with the axis of travel of the plunger.

The piston 510 includes a cavity 512 to receive the motor 506 and the lead screw 508 such that the lead screw 508 and the motor 506 are substantially contained within the piston cavity 512 when the piston 510 is in a retracted position. In this case, the piston 510 and lead screw 508 have a "telescoping" configuration, as will be described in more detail below. The piston 510 includes a cap 513, a first member 514 and a second member 516. The cap 513 is affixed to the first member 514. At least one spline 517 on an inner surface 519 of the first member 514 mates with at least one groove (not shown) on an outer surface of the second member 516. The at least one spline 517 prevents rotational movement of the first member 514 such that the first member 514 only moves in an axial direction A'. The second member 516 is at least partially slidably inserted into the first member 514 and includes internal threads 544 that mate with external threads 542 on the lead screw 508. The second member 516 includes a keying feature 518 (e.g., a flange) on a proximal end that mates with a slot (not shown) on an inner surface of the drug delivery device housing. The keying feature 518 prevents rotation of the second member such that the second member only moves in the axial direction A'.

In this example of the drive mechanism 500, the motor 506 is a "flat" motor with the diameter being greater than the length. The length of the motor is from about 2 millimeters to about 12 millimeters and the diameter of the motor is from about 10 millimeters to about 15 millimeters. The configuration of the piston 510, lead screw 508 and motor 506 results in a more compact drug delivery device than with conventional motor configurations, which are parallel to the axis of travel of the plunger.

The motor 506 drives a drive shaft 520, which is coupled to a drive nut 522. The motor 506 is housed within and is attached to a motor mounting sleeve 528. The motor mounting sleeve 528 prevents the motor 506 from rotating by being keyed (not shown) to a base mount 532 that is attached to an internal surface of the drug delivery device. The base mount 532 is nested inside the motor mounting sleeve 528 near the proximal end 534 of the motor mounting sleeve 528. A plurality of linear bearings 536 between the motor mounting sleeve 528 and the base mount 532 allow the motor mounting sleeve 528 to "float" axially such that a force sensor 538 can sense a load on the motor 506 when, for example, the infusion line that delivers the drug from the drug reservoir is occluded. The force sensor 538 is coupled to a force sensor contact 540 at the proximal end of the motor 506.

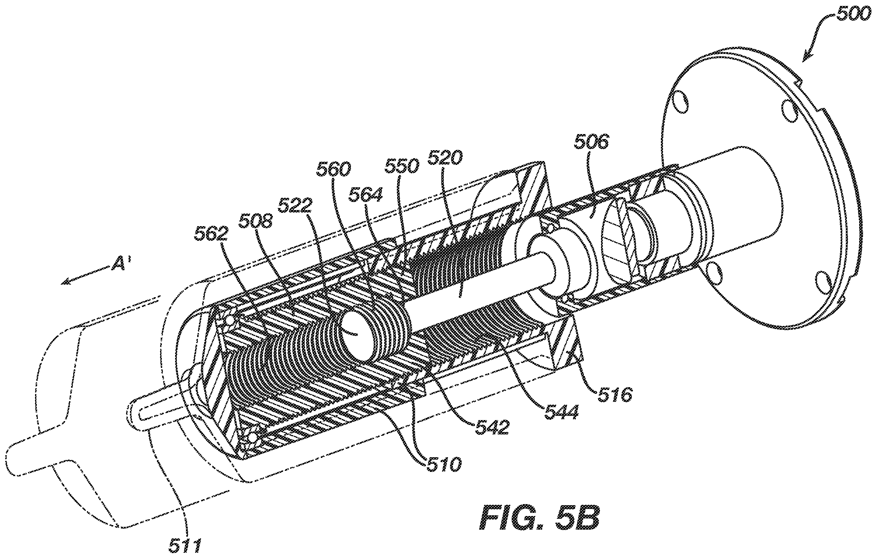

A distal end 535 of the motor mounting sleeve 528 is located adjacent to a second end 550 of the lead screw 508 when the piston 510 is in a retracted position. In order for the drive shaft 520 to connect to the drive nut 522, the drive shaft 520 protrudes through an opening 552 in the distal end 535 of the motor mounting sleeve 528. A first dynamic radial seal 554 is located between the drive shaft 520 and the motor mounting sleeve 528 to prevent fluid from contacting the motor 506. The first dynamic radial seal 554 allows axial movement of the motor mounting sleeve 528 for force sensing. The static radial seal 554 may be formed from a low friction material such as, for example, Teflon. As shown in FIGS. 5A and 5B, the drive nut 522 spans the longitudinal distance from the first end 526 to the second end 550 inside a lead screw cavity 556. In an alternative example, the drive nut 522 spans a portion of the distance from the first end 526 to the second end 550 inside the lead screw cavity 556 and the length of the drive shaft 520 is increased accordingly.

A dynamic radial seal 558 may also be located between the base mount 532 and the motor mounting sleeve 528 to prevent fluid from reaching the motor 506. The dynamic radial seal 558 allows axial movement of the motor mounting sleeve 528 for force sensing. The dynamic radial seal 558 may be formed from a low friction material such as, for example, Teflon.

The drive nut 522 includes external threads 560 that mate with internal threads 562 of the lead screw 508. The lead screw 508 also includes external threads 542 that mate with internal threads 544 of the second member 516 of the piston 510. Radial bearings 546 may be included in a space 548 between the first end 526 of the lead screw 508 and an inner surface of the first member 514 of the piston 510 to allow rotation of the lead screw 508.

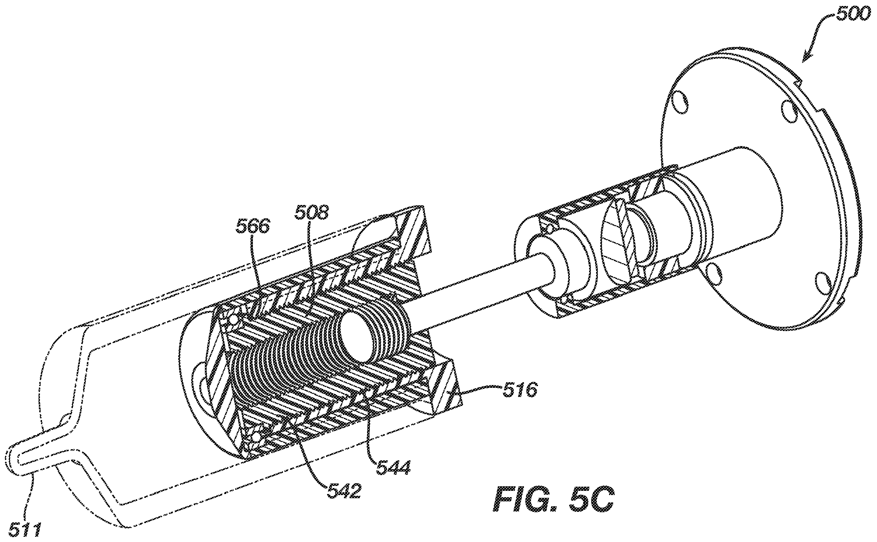

In use, the torque generated from the motor 506 is transferred to the drive shaft 520, which then rotates the lead screw 508. As the lead screw 508 rotates, the external threads 560 of the drive nut 522 engage with the internal threads 562 of the lead screw 508 such that the lead screw 508 moves first distance B1 in an axial direction until a first stop 564 on the drive nut 522 is engaged with an internal surface of the second end 550 of the lead screw 508, as illustrated in FIG. 5B. Because the external threads 542 near the second end 550 of the lead screw 508 are engaged with the internal threads 544 of the second member 516 of the piston 510 and the piston 510 can only move axially, the piston 510 also moves first distance B 1. Next, the external threads 542 of the lead screw 508 engage with the internal threads 544 of the second member 516 of the piston 510, causing the piston 510 to move a second distance B2 in an axial direction until a second stop 566 on an external surface of the lead screw 508 is engaged, as illustrated in FIG. 5C. Thus, the piston 510 moves from a retracted position (see FIG. 5A) to a fully extended (or telescoped) position (see FIG. 5C). As the piston 510 moves from the retracted to the extended position, the distal end of the piston 510 engages the plunger 511 such that the drug is delivered from the drug reservoir or cartridge. Because the internal and external threads of the components in the drive mechanism 500 have the same pitch, the order in which the components move axially is not critical to the function of the drive mechanism 500.

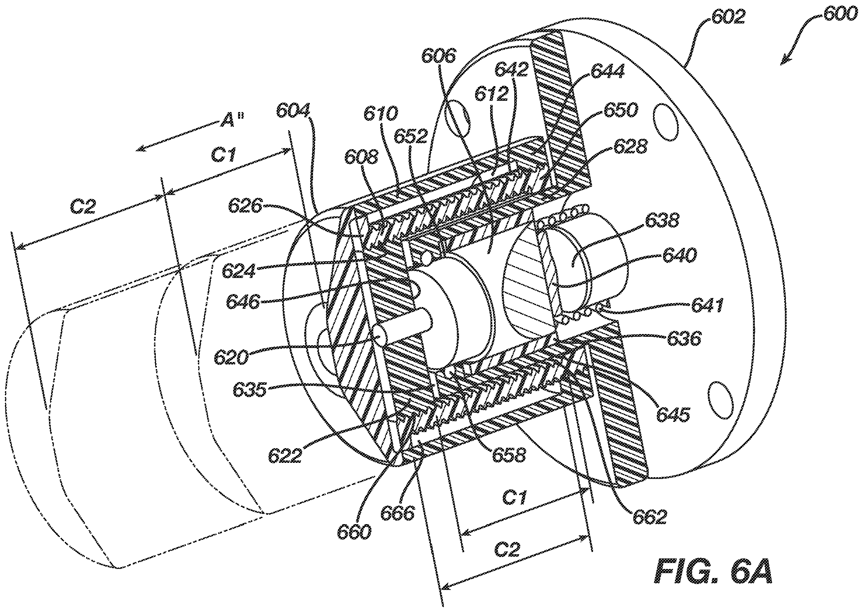

FIGS. 6A - 6C illustrate yet another example. The drive mechanism 600 is cylindrical in shape and includes a proximal end 602, a distal end 604 and a motor 606 operatively coupled to a lead screw 608 that is configured to engage a piston 610. The proximal end 602 of the drive mechanism 600 is compliance mounted to an internal surface (not shown) of a housing of a drug delivery device. The distal end 604 of the drive mechanism 600 is configured to engage a plunger (not shown) that is slidably inserted into a drug reservoir of a drug delivery device. The drive mechanism 600 is coaxially aligned or "in-line" with the axis of travel of the plunger.

The piston 610 includes a cavity 612 to receive the motor 606 and the lead screw 608 such that the lead screw 608 and the motor 606 are substantially contained within the piston cavity 612 when the piston 610 is in a retracted position. In this case, the piston 610 and lead screw 608 have a "telescoping" configuration, as will be described in more detail below. The piston 610 includes internal threads 644 near a proximal end that mate with external threads 642 on the lead screw 608. The piston 610 further includes a keying feature (not shown) on an outer surface of the proximal end that mates with a slot (not shown) on an inner surface of the drug delivery device housing. The keying feature prevents rotation of the piston 610 such that the piston 610 only moves in an axial direction A".

In this case, the motor 606 is a "flat" motor with the diameter being greater than the length. The length of the motor 606 is from about 2 millimeters to about 12 millimeters and the diameter of the motor 606 is from about 10 millimeters to about 15 millimeters. The configuration of the piston 610, lead screw 608 and motor 606 results in a more compact drug delivery device than with conventional motor configurations which are parallel to the axis of travel of the plunger.

The motor 606 is coupled to and drives a drive shaft 620. The drive shaft 620 is coupled to a drive nut 622 to an inner surface 624 of a first end 626 of the lead screw 608. The motor 606 is housed within a motor mounting sleeve 628, which prevents the motor 606 from rotating by being affixed (not shown) to an internal surface of the drug delivery device. A plurality of linear bearings 636 located between the motor 606 and the motor mounting sleeve 628 allow the motor 606 to "float" axially such that a force sensor 638 can sense a load on the motor 606 when, for example, the infusion line that delivers the drug from the drug reservoir is occluded. The force sensor 638 is coupled to a force sensor contact 640 at the proximal end of the motor 606. A spring 641 may optionally be located between the motor 606 and the drug delivery device housing such that the motor 606 is biased away from the force sensor 638.

A distal end 635 of the motor mounting sleeve 628 is located adjacent to a second end 646 of the drive nut 622 when the piston 610 is in a retracted position. In order for the drive shaft 620 to connect to the drive nut 622, the drive shaft 620 protrudes through an opening 652 in the distal end of the motor mounting sleeve 628. A dynamic radial seal 658 is located between the drive shaft 620 and the motor mounting sleeve 628 to prevent fluid from contacting the motor 606. The dynamic radial seal 658 allows axial movement of the motor mounting sleeve 628 for force sensing. The dynamic radial seal 658 is formed from a low friction material such as, for example, Teflon.

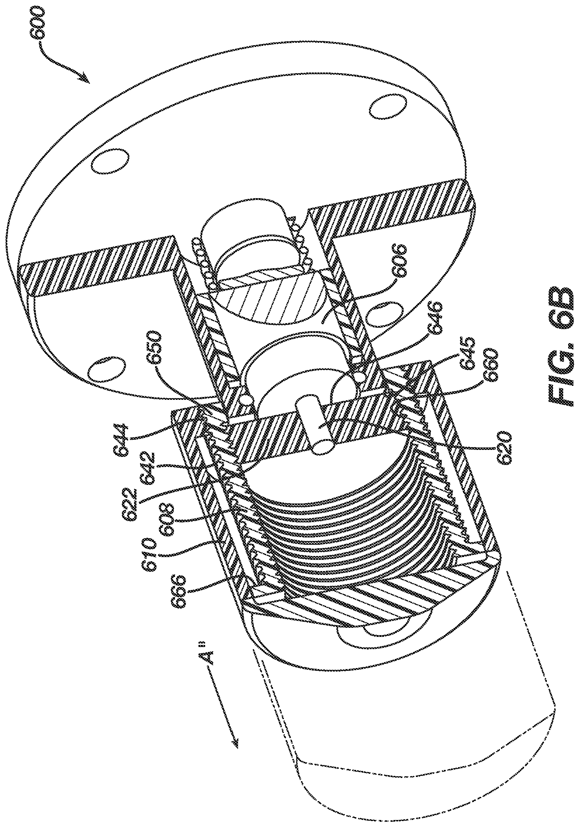

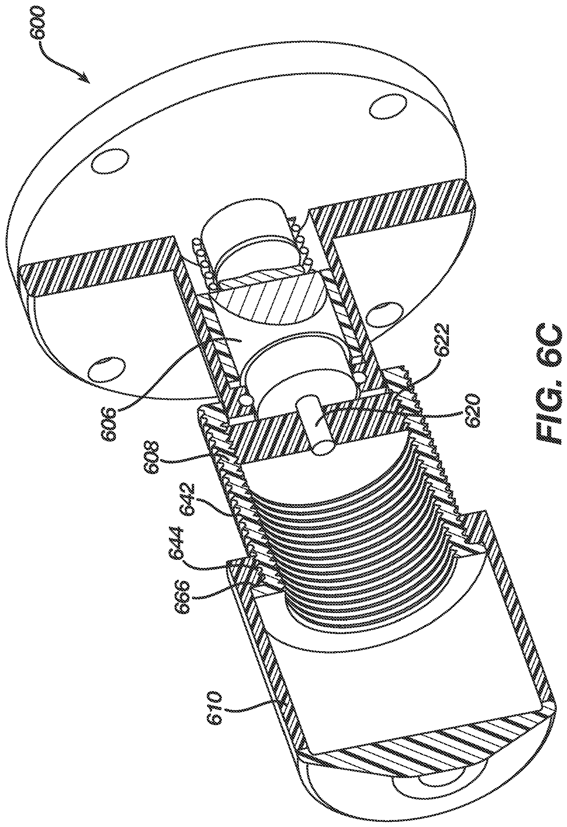

The drive nut 622 includes external threads 660 that mate with internal threads 662 of the lead screw 608. In use, the torque generated from the motor 606 is transferred to the drive shaft 620, which then rotates the lead screw 608. As the lead screw 608 rotates, the external threads 660 of the drive nut 622 engage with the internal threads 662 near the first end 626 of the lead screw 608 such that the lead screw 608 moves a first distance C1 in an axial direction until a surface 645 on the proximal end of the lead screw 608 engages the second end 646 of the drive nut 622 , as illustrated in FIG. 6B. Because the external threads 642 near the second end 650 of the lead screw 608 are engaged with the internal threads 644 of the piston 610 and the piston 610 can only move axially, the piston 610 also moves the first distance C1 in an axial direction. Next, the external threads 642 near the second end 650 of the lead screw 608 engage with the internal threads 644 near the proximal end of the piston 610, causing the piston 610 to move a second distance C2 in an axial direction until a stop 666 on an external surface of the lead screw 608 is engaged, as illustrated in FIG. 6C. Thus, the piston 610 moves from a retracted position (see FIG. 6A) to a fully extended (or telescoped) position (see FIG. 6C). As the piston 610 moves from the retracted to the extended position, the distal end of the piston 610 engages the plunger such that the drug is delivered from the drug reservoir or cartridge. Because the internal and external threads of the components in the drive mechanism 600 have the same pitch, the order in which the components move axially is not critical to the function of the drive mechanism 600.

An advantage of the telescoping arrangement illustrated in FIGS. 6A - 6C is that the length of the piston 610 can be reduced by about 40% (or distance C1 in FIG. 6A) versus non-telescoping configurations, resulting in a more compact drug delivery device.

The motors depicted in FIGS. 1 - 6B may optionally include an encoder (not shown) that, in conjunction with the electronics of the drug delivery device, can monitor the number of motor rotations. The number of motor rotation can then be used to accurately determine the position of the piston, thus providing information relating to the amount of fluid dispensed from the drug reservoir.

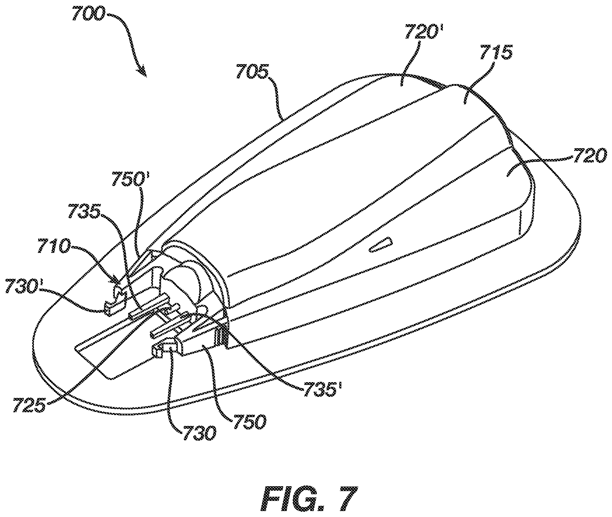

FIG. 7 illustrates an infusion device according to the present invention, employing an in-line drive mechanism. This case relates to an inline infusion pump with an adapter to permit it to be used as a hybrid device - either tethered or untethered. Many insulin pumps require the use of an infusion set that attaches to the reservoir or cartridge within the pump to deliver medication under the skin. Exemplary of such an infusion set is the one described in U.S. Patent No. 6,572,586.

Some patient may prefer having their infusion pump located remotely from their infusion site where the cannula of the infusion set is inserted under the skin. Those patients will prefer to use the presently disclosed infusion system with an infusion set. Others, however, choose to avoid the use of an infusion set and will opt for a patch-style (e.g. untethered) infusion pump. This style of infusion pump use omits the use of the infusion set and the cannula that is inserted under the skin of the user extends directly from the cartridge or reservoir of the infusion pump. A wearable, patch-style infusion device exemplary of untethered pumps is described in U.S. Patent No. 8,109,912.

The infusion device 700 includes housing 715 that contains within it the inline drive mechanism and cartridge, reservoir, bladder, or other structure for storing medication. The housing 715 includes flexible wings 720, 720' that are attached to the housing, but are made from a soft, pliant material, such as silicone rubber, that will allow the device to conform to the location on the patient's body where the device 700 is worn. The device 700 is adhered to the patient's body using an adhesive patch 705 that may be attached to the housing 715 via ultrasonic welding, laser welding, chemical bonding agents, etc.

Since devices are typically used by Type 1 diabetics, when the device is configured to deliver basal insulin, having a structure that permits the device to adhere securely and comfortably to the body of patients of varying sizes (children through adults) is beneficial. Using flexible wings 720, 720' on either side of the device 700 allows the device 700 to rest more securely against the contours of the body while reducing stresses at locations on the adhesive patch 705. This makes it less likely that a patient will accidentally dislodge their patch pump, whether through exercise, normal activity (walking, performing household chores, etc.), during movement while sleeping, etc. Patients should also find that a housing 715 with a semi-pliant design is more comfortable, as the likelihood of a sharp edge or corner protruding from the device and causing irritation or discomfort is minimized.

The infusion device 700 shown also has the ability to operate as a tethered pump, meaning that it uses an infusion set to connect the fluid outlet port 725 on the pump 700 to a cannula that is inserted under the skin of the patient at a remote location. Alternatively, the device 700 can operate as an untethered pump that has a cannula directly attached to the device's fluid output port 725 and be inserted under the skin of the patient at a location proximate to the location on the patient's body where the device 700 adheres via the adhesive patch 705.

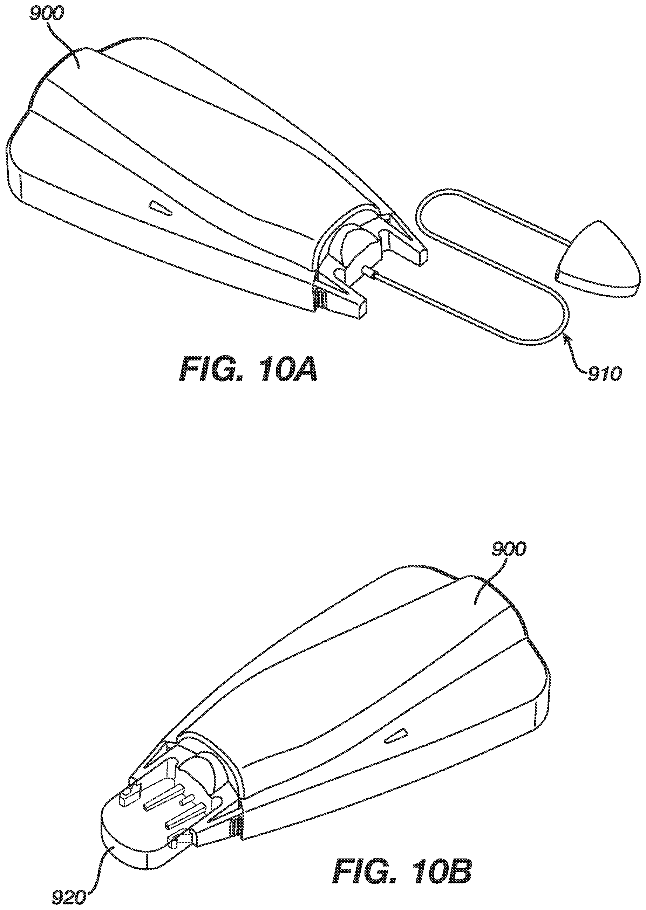

The device 700 includes a receiver mechanism 710 for receiving an infusion set or cannula that includes finger-press tabs 750, 750' that are used to deflect catch-tabs 730, 730' that releasably attaches to an infusion set or an cannula, as illustrated in FIGS 10A, 10B. Guide tabs 735, 735' help place the cannula adapter 920 (FIG. 10B) to ensure that the cannula is connected to the fluid outlet port 725. As shown in FIG. 10A, a hybrid pump housing with flexible wings 900 attaches to an infusion set 910. In FIG. 10B, the hybrid pump housing with flexible wings 900 attaches to a cannula adapter 920.

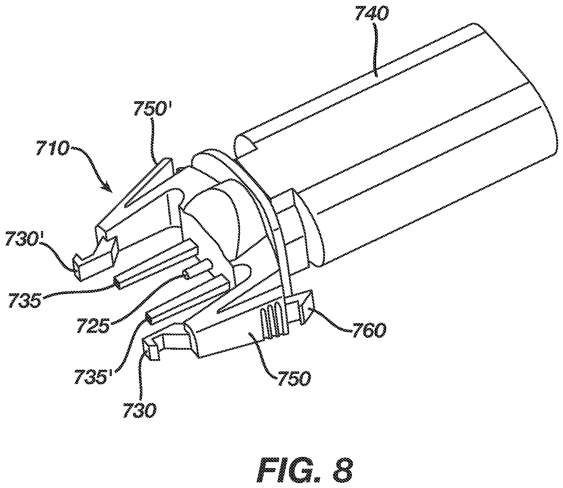

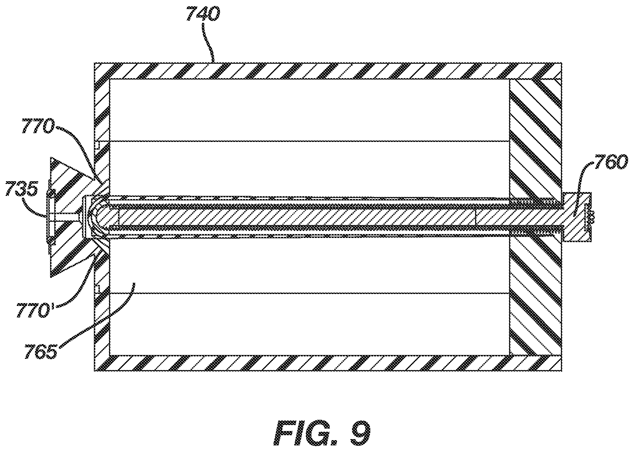

As further illustrated in FIG. 8, the receiver mechanism 710 also may include latch tabs 760 that releasably secure the receiver mechanism 710 to the housing 715. As illustratively shown in FIG. 8, the receiver mechanism 710 is attached to a housing insert 740. The housing insert 740, as illustratively shown in FIG. 9, may include an in-line drive mechanism 760, or other type of fluid pumping mechanism such as a peristaltic pump, micro-electrical mechanical pump (MEMS) or other drive system known in the art. In addition, the housing insert may include a reservoir for medication 765 that has fluid channels 770, 770' for communicating with the fluid outlet port 725. In one case, the reservoir 765 portion of the housing comprises a flexible bladder that fits within the flexible wings 720, 720'. Alternatively, the housing insert 740 may comprise the fluid drive mechanism 760 and a reservoir could be formed within cavities in the flexible wings 720, 720'.

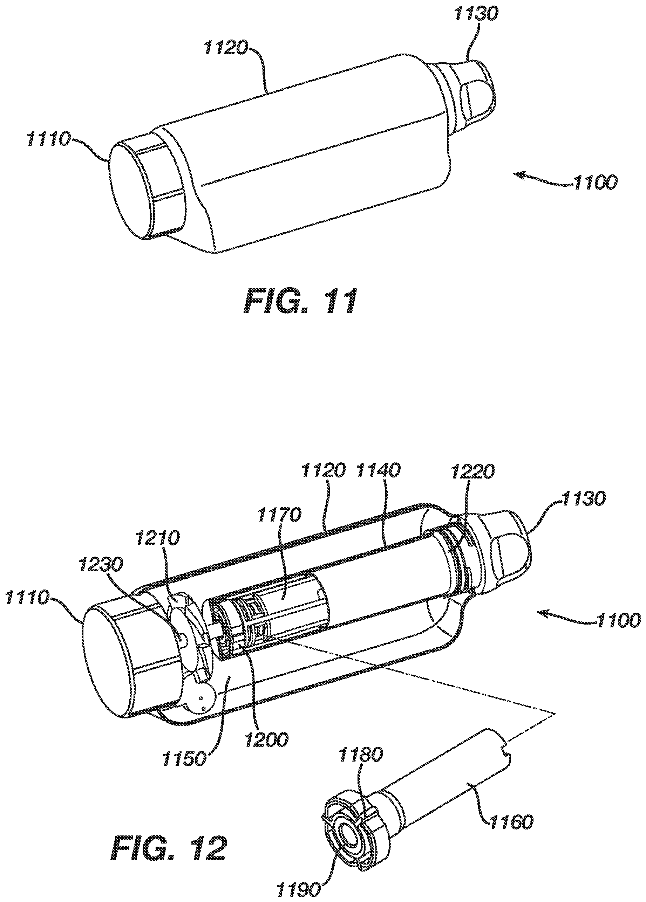

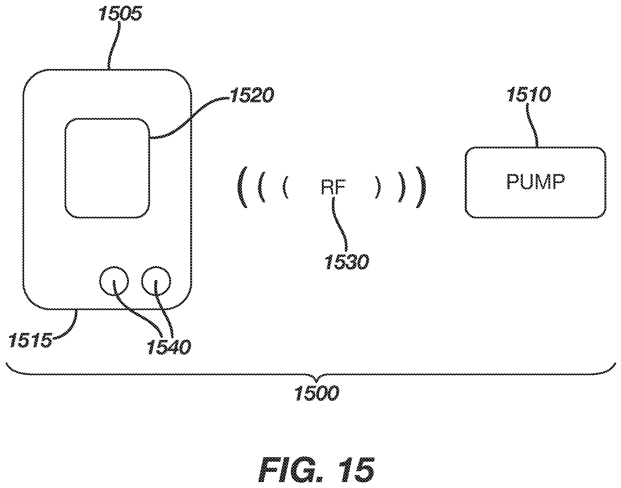

FIGS. 11 - 14 illustrate a bolus only pump that enables delivery of insulin via a mechanical drive that is controlled by the patient. Unlike other, purely mechanical pumps, this system incorporates a small amount of electronics to provide an RF link and a means of locking out the delivery mechanism. In this embodiment, the pump has no display or control buttons. This pump is configured to be operated by a remote controller, shown generally in FIG. 15, that can include SMBG (self-monitored blood glucose) to assist diabetic patients determine their needed dosage of, for example, insulin. Remote controllers suitable for use according to this embodiment of the invention are described more fully in U.S. Patents 8,449,523 and 8,444,595.

According to this embodiment of the present invention, when the patient needs a bolus of insulin, they enter the amount into the remote controller 1505 (FIG. 15) using input keys 1540. The amount can be confirmed on the display 1520 on the housing 1515 of the remote controller 1505. The remote controller 1505 connected to the pump 1510 via n RF link 1530 forms a remote controlled infusion system 1500. The remote controller 1505 sends a message to the pump 1510 via an RF (radio frequency) link 1530 that tells the pump to unlock the mechanical drive mechanism. While RF links are commonly used in the industry, such as Bluetooth®, infra-red (IR), and other methods and protocols for wireless telemetry can be used.

The patient then turns a dial a desired number of clicks to deliver the desired amount of medication. The rotary motion of the dial is translated into linear motion, driving a plunger within a standard barrel style cartridge. e.g. one click of the dial equals one unit of insulin. The pump counts the number of clicks to ensure the proper amount of medication is delivered. Once the desired amount is achieved, a locking mechanism engages, disabling further delivery of medication. If the patient needs more medication, they need to enter it through the remote. If the patient does not finish the delivery within a preset amount of time, a warning is displayed on their remote.

An embodiment of the present invention is illustrated in FIG. 11 in which a bolus-only medical infusion device 1100 has a housing 1120. An end cap 1130 located at a distal end of the housing 1120 secures a cartridge containing medication within the housing 1120. A dial 1110, located at a proximal end of the housing 1120, allows the patient, user, or healthcare provider to manual set the size of a bolus to be delivered.

FIG. 12 shows the housing 1120 with a conventional, barrel-style medicament cartridge 1140 disposed within the housing 1120. The medicament cartridge may include a sealing member 1220, such as a rubber o-ring, distal end of the housing to minimize or negate water, moisture, fluid, or contaminant incursion into the housing 1120. The cartridge cap 1130 may be removably attached to the housing 1120 to retain the cartridge 1140 securely therein. Alternative cartridge caps are described more fully in U.S. Patent 8,361,050.

The cartridge 1140 includes a plunger 1170 that fits within the barrel bore of the cartridge 1140 to expel fluid from the cartridge 1140 as the plunger 1170 is advanced. In order to advance the plunger 1170, a pusher rod 1160 biases against the plunger 1170. The pusher rod 1160 includes a threaded bushing 1190 and anti-rotation guides 1180. A motor 1200 drives a threaded axle (not shown) into the threaded bushing 1190. Thus, as the motor 1200 causes the threaded axle to rotate, the threaded bushing 1190 follows the threads of the threaded axle via the threaded bushing 1190 causing the pusher rod 1160 to move linearly and bias against the plunger 1170 to expel fluid from the cartridge 1140.

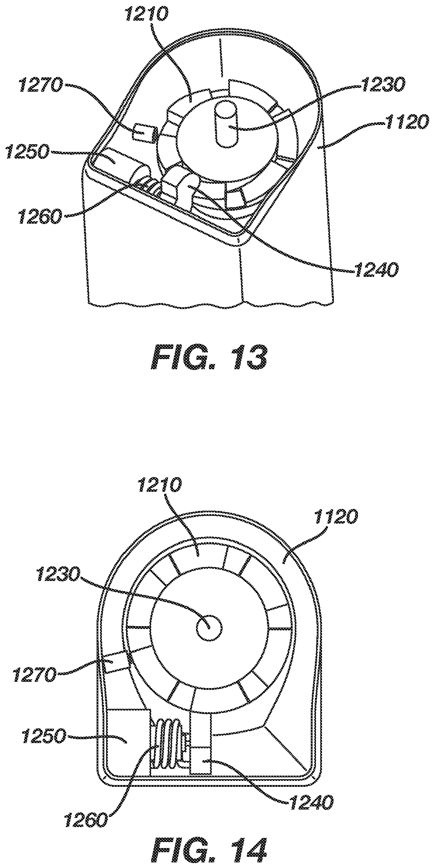

In order to determine the size of the bolus of medication to be delivered, the infusion device 1100 includes a dial 1110. When the dial 1110 is turned, a control axle 1230 depending from the dial 1110 and connecting to a control gear 1210 turns the control gear 1210. As shown in FIG. 13, a ratchet claw 1240 engages the control gear 1210, creating an audible "click" each time the ratchet claw 1240 passes a ramped tooth of the control gear 1210. Each "click" indicates a single unit of measure, such as 1 unit, 1 ml, etc. to be added to the bolus. If the patient turns the dial 1110 until three "clicks" are heard, the bolus size will be set for three times the base unit of measurement for the device, such as 3 units of fluid to be delivered when the device is actuated.

Inside the housing 1120, a motor 1250 and spring 1260 are provided to hold the ratchet claw 1240. As shown in FIG. 14, one or more sensors 1270 can be placed around the control gear 1210 to relay information to a control unit regarding the exact location of the control gear at any time.

Notable is that the device of this embodiment of the invention does not include any control buttons, display screens, etc. on or integral to the housing 1120 of the device 1100. Instead, a power supply, microprocessor or microcontroller, and telemetry system may be included in the housing 1120 in a cavity 1150 reserved for the electronic control system and power needed for motors 1250 and 1200.

Hand-held remote controls compatible with this embodiment of the invention were previously described. In this embodiment, the remote control unit is used to actuate delivery of medication. As was previously described, when the patient needs a bolus of insulin, they enter the amount into their remote device. This device sends a message to the pump via an RF (radio frequency) link that tells the pump to unlock the mechanical drive mechanism by disengaging the ratchet claw 1240 from the control gear 1210. This permits the control gear 1210 to turn.

In an embodiment that does not require the motor 1200, the patient turns the dial 1110 a desired number of "clicks" once the ratchet claw 1240 is disengaged, causing the control gear 1210 to rotate. In this embodiment, the control gear 1210 is directly linked to the threaded rod (not shown). As the user turns the dial 1110, the rotation of the threaded rod in the threaded bushing 1190 causes the pusher rod 1160 to move linearly and bias the plunger 1170 into the cartridge 1140 to expel fluid. Once the amount of medication programmed into the remote has been manually delivered by the patient by turning the dial 1110 the corresponding number of "clicks", a locking mechanism engages by the controller instructing the motor 1250 to re-engage the ratchet claw 1240 with the control gear 1210, disabling further delivery of medication. If the patient wishes to deliver medication, they need to enter it through the remote. If the patient does not finish the delivery within a preset amount of time, a warning is displayed on their remote and the locking mechanism may re-engage.

After a number of deliveries, the supply of medication in the cartridge 1140 will be exhausted. When the cartridge 1140 is empty the dial 1110 will not be able to turn any further, as the plunger 1170 will be fully extended into the cartridge 1140. The patient then rewinds the drive mechanism by turning the dial 1110 counterclockwise until it reaches the beginning of the stroke. Although it is not shown in the drawing figures, this process can be made simple and quick by adding a quick nut or educated nut to enable a quick release of the threaded bushing 1190 from the threaded rod. For example, a button on the quick nut is pushed, which disengages the threads and allows the drive mechanism to slide back quickly rather than turning the dial 1110 through multiple rotations to get back to the starting position.

At least two implementations of the 'quick release' button can be accomplished. The first would have the button of the quick nut exposed along one side of the infusion device 1100. The button may ride in a slot that is as long as the stroke of the plunger 1170. When a rewind needed to occur, the patient would simultaneously push the button in and slide it toward the dial 1110. Once the button is released the threads would reengage. A second configuration would have the release button in the center and on top of the delivery dial 1110. This would require more intricate mechanics to push the release button on the quick nut, but it would allow for more easily avoid water or moisture incursion into the device.

Upon completion of the rewind, the remote control 1505, as illustrated in FIG. 15, can be notified that the system is in the home position due to sensors 1270. The system 1500 could then calculate the amount of medication remaining based on the starting position of the drive when a filled medication cartridge is inserted into the infusion device 1000 and/or 1510. Additional position sensors could be added in the housing 1120 to provide greater resolution of the plunger 1170 position, thus greater accuracy with respect to the quantity of medication in the cartridge 1140. A viewing window may be added to the housing 1120, so the patient can see how much insulin is remaining as well.

It will be recognized that equivalent structures may be substituted for the structures illustrated and described herein and that the described embodiment of the invention is not the only structure, which may be employed to implement the claimed invention. In addition, it should be understood that every structure described above has a function and such structure can be referred to as a means for performing that function. While embodiments of the present invention have been shown and described herein, it will be obvious to those skilled in the art that such embodiments are provided by way of example only. Numerous variations, changes, and substitutions will now occur to those skilled in the art without departing from the invention.

It should be understood that various alternatives to the embodiments of the invention described herein may be employed in practicing the invention. It is intended that the following claims define the scope of the invention and that structures within the scope of these claims be covered thereby.

## Claims

1. A medical infusion device (1100), comprising:
a housing (1120) having proximal end, a distal end, and a cavity therein;
an opening at the distal end of the housing for receiving a medicament cartridge (1140) into the cavity, wherein the medicament cartridge (1140) comprises a cylindrical housing having an open proximal end and a distal end configured to detachably connect to luer, and a plunger (1170) configured for insertion into the proximal end of the cylindrical housing; and
a pusher rod (1160) configured to bias the plunger (1170) into the cylindrical housing of the medicament cartridge (1140), the pusher rod (1160) comprising at least one anti-rotation guide (1180) and a threaded bushing (1190);
**characterized by:**
a threaded axle configured so that the threaded axle screwably engages with the threaded bushing (1190), thereby inducing linear motion of the pusher rod (1160) when the threaded axle rotates;
a control gear (1210) mechanically linked to the threaded axle;
a dial (1110) mechanically linked to the control gear (1210); and
a ratchet claw (1240) configured for releasable engagement of the control gear (1210) to inhibit rotation of the control gear (1210) when the ratchet claw (1240) is engaged,
wherein the ratchet claw (1240) is releasably engaged by a remote controller (1505) in wireless communication with the medical infusion device (1100).

2. The medical infusion device (1100) of claim 1 wherein the ratchet claw (1240) is disengaged from the control gear (1210) to permit rotation of the dial (1110).

3. The medical infusion device (1100) of claim 2 wherein the control gear (1210) comprises ramped teeth that permit the dial (1110) to be turned in discrete increments.

4. The medical infusion device (1100) of claim 3 wherein each discrete increment corresponds to a discrete amount of linear movement of the pusher rod (1160).

5. The medical infusion device (1100) of claim 4 comprising a motor (1250) and a torsion spring (1260) in mechanical linkage with the ratchet claw (1240).

6. The medical infusion device (1100) of claim 5 comprising an RF receiver in electrical communication with the motor (1250).

7. The medical infusion device (1100) of claim 6 comprising a remote controller (1505) configured for RF communication with the RF receiver.

8. The medical infusion device (1100) of claim 7 wherein the remote controller (1505) comprises a display screen (1520) and at least one data input key (1540).

9. The medical infusion device (1100) of claim 1 comprising one or more sensors (1270) for sensing the position of at least one of the control gear (1210), the pusher rod (1160), and the plunger (1170).

10. The medical infusion device (1100) of claim 8 wherein a user enters a desired dosage using the at least one data input key (1540) on the remote controller (1505).

11. The medical infusion device (1100) of claim 10 wherein the controller (1505) disengages the ratchet claw (1240) in response to the desired dosage being entered on the remote controller (1505).

12. The medical infusion device (1100) of claim 11 wherein the dial (1110) can be turned in a number of discrete increments equal to the desired dosage.

13. The medical infusion device (1100) of claim 12 wherein the remote controller (1505) engages the ratchet claw (1240) after the dial (1110) is turned in the number of discrete increments equal to the desired dosage.

14. The medical infusion device (1100) of claim 13 wherein the at least one sensor (1270) is configured to send an RF signal to the remote controller (1505) when the plunger (1170) is biased by the pusher rod (1160) to a predefined position within the medicament cartridge (1140).

15. The medical infusion device (1100) of claim 1 wherein the anti-rotation guide (1180) prohibits rotation of the pusher rod (1160) when the threaded axle is rotating.

## Patentansprüche

1. Medizinische Infusionsvorrichtung (1100), umfassend:
ein Gehäuse (1120) mit einem proximalen Ende, einem distalen Ende und einem darin befindlichen Hohlraum;
eine Öffnung am distalen Ende des Gehäuses zum Aufnehmen einer Medikamentenpatrone (1140) in den Hohlraum, wobei die Medikamentenpatrone (1140) ein zylindrisches Gehäuse mit einem offenen proximalen Ende und einem distalen Ende, das derart konfiguriert ist, dass es lösbar mit einem Luer-Anschluss verbunden ist, und einen Kolben (1170), der zum Einführen in das proximale Ende des zylindrischen Gehäuses konfiguriert ist, umfasst; und
eine Schubstange (1160), die derart konfiguriert ist, dass sie den Kolben (1170) in das zylindrische Gehäuse der Medikamentenpatrone (1140) vorspannt, wobei die Schubstange (1160) mindestens eine Verdrehsicherung (1180) und eine mit einem Gewinde versehene Buchse (1190) umfasst;
**gekennzeichnet durch**:
eine mit einem Gewinde versehene Achse, die derart konfiguriert ist, dass die mit einem Gewinde versehene Achse schraubbar mit der mit einem Gewinde versehenen Buchse (1190) in Eingriff kommt, wodurch eine lineare Bewegung der Schubstange (1160) induziert wird, wenn sich die mit einem Gewinde versehene Achse dreht;
ein mit der mit einem Gewinde versehenen Achse mechanisch verbundenes Steuerzahnrad (1210);
eine mit dem Steuerzahnrad (1210) mechanisch verbundene Wählscheibe (1110); und
eine Sperrklinke (1240), die zum lösbaren Eingriff des Steuerzahnrads (1210) konfiguriert ist, um eine Drehung des Steuerzahnrads (1210) zu verhindern, wenn die Sperrklinke (1240) in Eingriff ist,
wobei die Sperrklinke (1240) **durch** eine Fernbedienung (1505) in drahtloser Kommunikation mit der medizinischen Infusionsvorrichtung (1100) lösbar in Eingriff gebracht wird.

2. Medizinische Infusionsvorrichtung (1100) nach Anspruch 1, wobei die Sperrklinke (1240) von dem Steuerzahnrad (1210) außer Eingriff gebracht wird, um eine Drehung der Wählscheibe (1110) zu ermöglichen.

3. Medizinische Infusionsvorrichtung (1100) nach Anspruch 2, wobei das Steuerzahnrad (1210) geneigte Zähne aufweist, die es der Wählscheibe (1110) ermöglichen, in einzelnen Schritten gedreht zu werden.

4. Medizinische Infusionsvorrichtung (1100) nach Anspruch 3, wobei jeder einzelne Schritt einer einzelnen Menge an linearer Bewegung der Schubstange (1160) entspricht.

5. Medizinische Infusionsvorrichtung (1100) nach Anspruch 4, die einen Motor (1250) und eine Torsionsfeder (1260) in mechanischer Verbindung mit der Sperrklinke (1240) umfasst.

6. Medizinische Infusionsvorrichtung (1100) nach Anspruch 5, die einen HF-Empfänger in elektrischer Verbindung mit dem Motor (1250) umfasst.

7. Medizinische Infusionsvorrichtung (1100) nach Anspruch 6, die eine Fernsteuerung (1505) umfasst, die zur HF-Kommunikation mit dem HF-Empfänger konfiguriert ist.

8. Medizinische Infusionsvorrichtung (1100) nach Anspruch 7, wobei die Fernbedienung (1505) einen Anzeigebildschirm (1520) und mindestens eine Dateneingabetaste (1540) umfasst.

9. Medizinische Infusionsvorrichtung (1100) nach Anspruch 1, umfassend einen oder mehrere Sensoren (1270) zur Abtastung der Position von wenigstens einem aus dem Steuerzahnrad (1210), der Schubstange (1160) und dem Kolben (1170).

10. Medizinische Infusionsvorrichtung (1100) nach Anspruch 8, wobei ein Benutzer eine gewünschte Dosierung unter Verwendung der zumindest einen Dateneingabetaste (1540) auf der Fernsteuerung (1505) eingibt.

11. Medizinische Infusionsvorrichtung (1100) nach Anspruch 10, wobei die Steuerung (1505) die Sperrklinke (1240) in Reaktion auf die Eingabe der gewünschten Dosierung auf der Fernbedienung (1505) außer Eingriff bringt.

12. Medizinische Infusionsvorrichtung (1100) nach Anspruch 11, wobei die Wählscheibe (1110) in einer Anzahl einzelner Schritte, die der gewünschten Dosierung entsprechen, gedreht werden kann.

13. Medizinische Infusionsvorrichtung (1100) nach Anspruch 12, wobei die Fernbedienung (1505) die Sperrklinke (1240) in Eingriff nimmt, nachdem die Wählscheibe (1110) in der Anzahl der einzelnen Schritte, die der gewünschten Dosierung entspricht, gedreht wird.

14. Medizinische Infusionsvorrichtung (1100) nach Anspruch 13, wobei der mindestens eine Sensor (1270) derart konfiguriert ist, dass er ein HF-Signal an die Fernsteuerung (1505) sendet, wenn der Kolben (1170) durch die Schubstange (1160) zu einer vordefinierten Position innerhalb der Medikamentenpatrone (1140) vorgespannt ist.

15. Medizinische Infusionsvorrichtung (1100) nach Anspruch 1, wobei die Verdrehsicherung (1180) eine Drehung der Schubstange (1160) verhindert, wenn sich die mit einem Gewinde versehene Achse dreht.

## Revendications

1. Dispositif d'infusion médical (1100), comprenant :
un boîtier (1120) ayant une extrémité proximale, une extrémité distale et une cavité en son sein ;
une ouverture au niveau de l'extrémité distale du boîtier pour recevoir une cartouche de médicament (1140) dans la cavité, dans lequel la cartouche de médicament (1140) comprend un boîtier cylindrique ayant une extrémité proximale ouverte et une extrémité distale configurée pour être raccordée de manière amovible à un raccord luer, et un piston plongeur (1170) configuré pour insertion dans l'extrémité proximale du boîtier cylindrique ; et
une tige de poussée (1160) configurée pour solliciter le piston (1170) dans le boîtier cylindrique de la cartouche de médicament (1140), la tige de poussée (1160) comprenant au moins un guide antirotation (1180) et une bague filetée (1190) ;
**caractérisé par** :
un axe fileté configuré de telle sorte que l'axe fileté vienne se visser sur la bague filetée (1190), ce qui permet de provoquer un mouvement linéaire de la tige de poussée (1160) lorsque l'axe fileté tourne ;
un mécanisme de commande (1210) relié mécaniquement à l'axe fileté ;
un bouton de réglage (1110) relié mécaniquement au mécanisme de commande (1210) ; et
une griffe d'encliquetage (1240) configurée pour permettre une mise en prise de manière amovible du mécanisme de commande (1210) pour empêcher la rotation du mécanisme de commande (1210) lorsque la griffe d'encliquetage (1240) est mise en prise,
dans lequel la griffe d'encliquetage (1240) est mise en prise de manière amovible par un dispositif de commande à distance (1505) en communication sans fil avec le dispositif d'infusion médical (1100).

2. Dispositif d'infusion médical (1100) selon la revendication 1, dans lequel la griffe d'encliquetage (1240) est désolidarisée du mécanisme de commande (1210) pour permettre une rotation du bouton de réglage (1110).

3. Dispositif d'infusion médical (1100) selon la revendication 2, dans lequel le mécanisme de commande (1210) comprend des dents inclinées qui permettent au bouton de réglage (1110) d'être tourné selon des incréments discrets.

4. Dispositif d'infusion médical (1100) selon la revendication 3, dans lequel chaque incrément discret correspond à une quantité discrète de mouvement linéaire de la tige de poussée (1160).

5. Dispositif d'infusion médical (1100) selon la revendication 4, comprenant un moteur (1250) et un ressort de torsion (1260) en liaison mécanique avec la griffe d'encliquetage (1240).

6. Dispositif d'infusion médical (1100) selon la revendication 5, comprenant un récepteur RF en communication électrique avec le moteur (1250).

7. Dispositif d'infusion médical (1100) selon la revendication 6, comprenant un dispositif de commande à distance (1505) configuré pour établir une configuration RF avec le récepteur RF.

8. Dispositif d'infusion médical (1100) selon la revendication 7, dans lequel le dispositif de commande à distance (1505) comprend un écran de visualisation (1520) et au moins une touche de saisie de données (1540).

9. Dispositif d'infusion médical (1100) selon la revendication 1, comprenant un ou plusieurs capteurs (1270) pour détecter la position du mécanisme de commande (1210) et/ou de la tige de poussée (1160) et/ou du piston plongeur (1170).

10. Dispositif d'infusion médical (1100) selon la revendication 8, dans lequel un utilisateur entre un dosage souhaité à l'aide de la ou des touches de saisie de données (1540) sur le dispositif de commande à distance (1505).

11. Dispositif d'infusion médical (1100) selon la revendication 10, dans lequel le dispositif de commande (1505) désolidarise la griffe d'encliquetage (1240) à la suite de l'entrée du dosage souhaité sur le dispositif de commande à distance (1505).

12. Dispositif d'infusion médical (1100) selon la revendication 11, dans lequel le bouton de réglage (1110) peut être tourné selon un nombre d'incréments discrets égal au dosage souhaité.

13. Dispositif d'infusion médical (1100) selon la revendication 12, dans lequel le dispositif de commande à distance (1505) vient en prise avec la griffe d'encliquetage (1240) après que le bouton de réglage (1110) est tourné selon le nombre d'incréments discrets égal au dosage souhaité.

14. Dispositif d'infusion médical (1100) selon la revendication 13, dans lequel le ou les capteurs (1270) sont configurés pour envoyer un signal RF au dispositif de commande à distance (1505) lorsque le piston plongeur (1170) est sollicité par la tige de poussée (1160) jusqu'à une position prédéfinie dans la cartouche de médicament (1140).

15. Dispositif d'infusion médical (1100) selon la revendication 1, dans lequel le guide antirotation (1180) empêche la rotation de la tige de poussée (1160) lorsque l'axe fileté tourne.
